Europäisches Patentamt

European Patent Office

Office européen des brevets

(19)

(11) Numéro de publication : **0 344 229 B1**

# (12) FASCICULE DE BREVET EUROPEEN

(45) Date de publication du fascicule du brevet :
**18.03.92 Bulletin 92/12**

(51) Int. Cl.⁵ : **A61F 13/15**

(21) Numéro de dépôt : **88908254.1**

(22) Date de dépôt : **23.09.88**

(86) Numéro de dépôt international :
**PCT/FR88/00469**

(87) Numéro de publication internationale :
**WO 89/02729 06.04.89 Gazette 89/08**

(54) **SERVIETTE PERIODIQUE OU SIMILAIRE A VOLETS LATERAUX IMPERMEABLES AUX FLUIDES.**

(30) Priorité : **25.09.87 FR 8713256**

(43) Date de publication de la demande :
**06.12.89 Bulletin 89/49**

(45) Mention de la délivrance du brevet :
**18.03.92 Bulletin 92/12**

(84) Etats contractants désignés :
**AT BE CH DE FR GB IT LI LU NL SE**

(56) Documents cités :
**EP-A- 0 121 966**
**FR-A- 2 494 226**
**US-A- 4 608 047**

(73) Titulaire : **KAYSERSBERG SA**
**Route de Lapoutroie**
**F-68240 Kaysersberg (FR)**

(72) Inventeur : **PIGNEUL, Raymond**
**2, rue des Vosges**
**F-68320 Durrenentzen (FR)**

(74) Mandataire : **David, Daniel et al**
**KAYSERSBERG Service Propriété Industrielle**
**23 boulevard Georges Clemenceau**
**F-92402 Courbevoie Cédex (FR)**

EP 0 344 229 B1

## Description

La présente invention concerne essentiellement un dispositif formant serviette périodique ou similaire à volets latéraux imperméables aux fluides de dimension suffisante pour être rabattus autour du linge de corps porté par la personne, ces volets latéraux étant avantageusement à utiliser, après emploi de la serviette périodique, pour former un sachet jetable par repliement, ces volets latéraux constituant de préférence partie intégrante d'une pochette de protection.

On connaît déjà par le document EP 0134086 une serviette périodique ou similaire, en particulier pour l'hygiène féminine, comprenant un élément tampon 216 absorbant les fluides disposé entièrement à l'intérieur d'une enveloppe 214-230-234 dont au moins la partie 214 de réception des fluides est perméable aux fluides. Des volets latéraux 224, 224' sont prévus pour améliorer l'étanchéité latérale par le fait que ces volets latéraux sont disposés à l'intérieur du linge de corps et ont leurs extrémités repliées contre le bord intérieur 246 ou 246' de ce linge de corps.

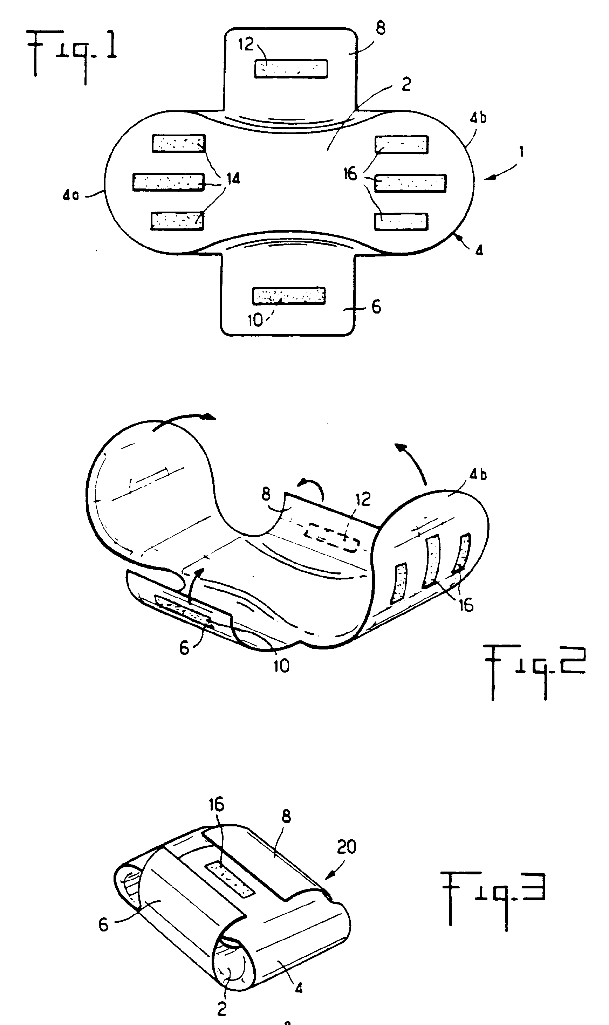

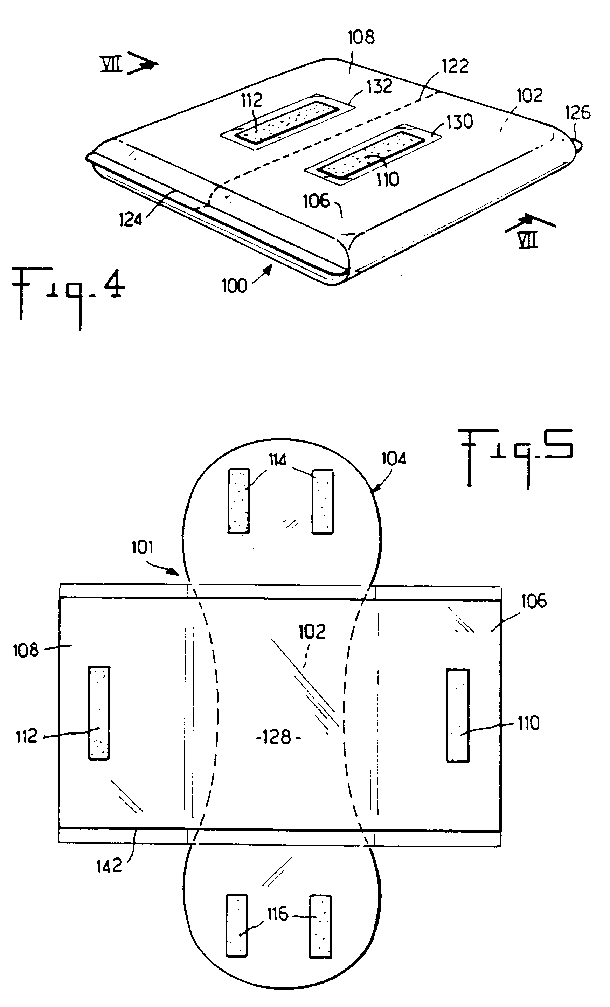

Il a pu être observé que ces volets latéraux étaient d'une dimension insuffisante pour protéger correctement l'ensemble du linge de corps. Ces volets latéraux peuvent également comporter des moyens de fixation amovibles, tels que pavés d'adhésifs pour assurer une fixation amovible sur le linge de corps, comme cela est clairement visible (figures 2, 4 et 7).

Le document EP-A 0130848 décrit un enseignement similaire, selon lequel en outre les volets latéraux peuvent également contenir à l'intérieur un élément tampon absorbant les fluides.

Des volets latéraux sont également décrits dans le document FR-A 2455885. Un autre enseignement similaire est décrit dans US-A 4285343, les volets latéraux étant rabattus extérieurement au linge de corps, le recouvrant complètement par une disposition en chevauchement (figures 2 ou 5) ou bord à bord (figure 7) tout en comportant encore une matière absorbante.

Le document US-A 4608047 décrit encore une serviette périodique comportant des volets latéraux de dimensions suffisantes pour pouvoir être rabattus extérieurement autour du linge de corps, tel que slip, porté par la personne, au moins un de ces volets latéraux comportant des moyens 34 de fixation amovibles des volets extérieurement sur ledit linge de corps, en obtenant ainsi une protection complète dudit linge de corps.

Par ailleurs, il est connu par le document FR-A 2494226 une structure pour l'emballage individuel d'articles d'hygiène, dont des serviettes périodiques, avant usage, ainsi qu'un procédé d'emballage au moyen de cette structure.

En outre, les serviettes périodiques une fois usagées sont en général jetées en étant introduites dans un sachet jetable livré séparément avec les serviettes.

Ceci constitue une complication d'emploi, et augmente les coûts de fabrication.

La présente invention a donc pour but de résoudre le problème technique consistant en la fourniture d'une solution permettant d'éviter l'emploi d'emballage distinct pour jeter la serviette périodique après usage.

La présente invention a encore pour but de résoudre le problème technique consistant en la fourniture d'une solution de dispositif formant serviette périodique ou similaire permettant d'éviter l'emploi d'un conditionnement individuel séparé indépendant.

Ces problèmes techniques sont résolus pour la première fois avantageusement simultanément, par la présente invention, d'une matière particulièrement simple, peu coûteuse, utilisable à l'échelle industrielle.

Ainsi, la présente invention fournit une serviette périodique ou similaire, en particulier pour l'hygiène féminine, comprenant un élément tampon absorbant les fluides disposé entièrement à l'intérieur d'une enveloppe dont au moins la partie de réception des fluides est perméable aux fluides, des volets latéraux étant prévus pour améliorer l'étanchéité latérale, lesdits volets latéraux étant de dimensions suffisantes pour pouvoir être rabattus extérieurement autour du linge de corps, tel que slip, porté par la personne, ces volets latéraux comprenant au moins un moyen de fixation amovible au linge de corps en obtenant ainsi une protection complète dudit linge de corps, lesdits volets latéraux étant conçus de façon à former, après emploi de la serviette périodique, par repliement autour de la serviette périodique elle-même repliée, un conditionnement ou sachet jetable est caractérisé en ce que les volets latéraux sont formés par une bande en matériau imperméable aux fluides, rapportée sur la serviette proprement dite et sont également conçus pour former partie intégrante d'une pochette de protection dans laquelle est conditionnée la serviette périodique avant emploi, la pochette étant obtenue d'une part par scellerent des volets à la partie centrale de la bande le long des bords perpendiculaires à l'axe de pliage des volets. d'autre part par scellement des volets l'un vers l'autre, parallèlement à cet axe, et comportant des moyens, de prédécoupe notamment, permettant de libérer les volets latéraux lors de l'ouverture de la pochette de protection.

La solution proposée par l'invention permet ainsi la réalisation d'un emballage de serviette parfaitement hermétique assurant la protection du tarpon contre la pénétration des poussières ou autres salissures avant emploi. Cet emballage est en outre particulièrement économique car il constitue un élément même de la serviette.

Selon un rode de réalisation, l'enveloppe précitée dans laquelle est disposé l'élément tarpon absorbant les fluides comporte. extérieurement à ses extrémités antérieure et postérieure, des moyens de solidarisation provisoire avec le linge de corps qui servent également au maintien de la serviette périodique en position repliée. Les moyens de fixation des volets sont eux avantageusement disposés vers leurs bords extérieurs, on obtient ainsi une disposition en croix de l'ensemble des moyens de fixation et de solidarisation.

Selon un rode de réalisation particulier, les moyens de fixation des volets latéraux sont disposés sur un seul des deux volets, sur la face extérieure du volet vers son extrémité libre, en permettant une fixation par chevauchement des extrémités des volets.

Selon un autre mode de réalisation particulier, les moyens de fixation des volets sont prévus au niveau de chaque extrémité des volets latéraux sur la paroi extérieure apparente des volets en étant protégés par des moyens de protection contre une destruction prématurée, amovibles, permettant la formation d'une pochette de protection soit par chevauchement, soit bord à bord.

Les moyens de protection contre une destruction prématurée, amovibles, peuvent être constitués par une bande siliconée, comme cela est classique.

On conçoit ainsi que l'on obtient tous les avantages techniques déterminants précédemment énoncés, à savoir une serviette périodique unique, comportant des volets latéraux conçus pour constituer un conditionnement, jetable après emploi, ces volets latéraux étant également conçus pour constituer au départ une pochette de protection de la serviette périodique. Enfin, ces volets latéraux servent également à la fixation complémentaire de la serviette périodique sur le linge de corps tout en le protégeant complètement durant l'emploi.

D'autres buts, caractéristiques et avantages de l'invention apparaîtront clairement à la lumière de la description explicative qui va suivre faite en référence aux dessins annexés représentant diverses variantes de réalisation de la serviette périodique selon l'invention, donnée simplement à titre d'illustration et quine saurait donc en aucune façon limiter la portée de l'invention. Dans les dessins :

– La figure 1 est une vue de dessous d'une serviette périodique comportant des volets latéraux ;

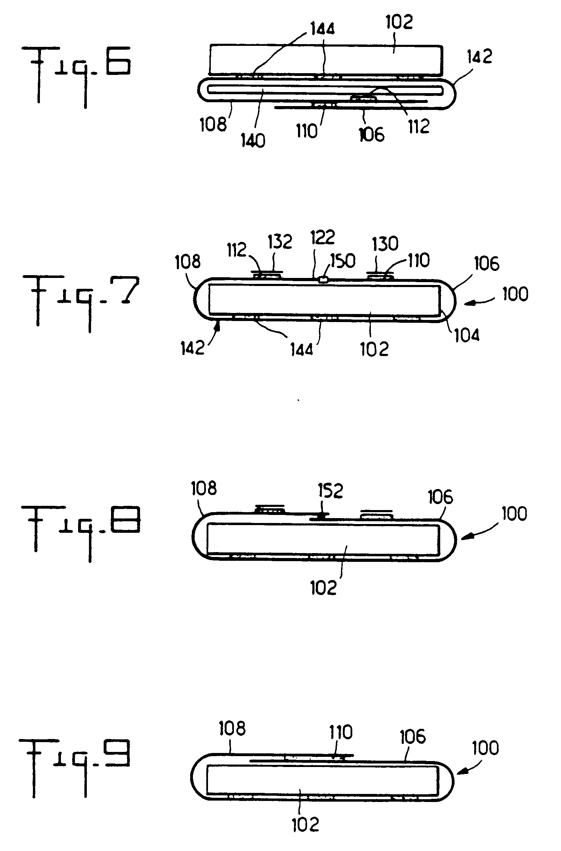

– La figure 2 représente schématiquement comment la serviette périodique est repliée sur elle-même, ainsi que les mouvements de repliement des volets latéraux de manière à constituer après repliement un conditionnement ou sachet jetable qui est représenté à la figure 3 ;

– La figure 3 est une vue schématique du dispositif replié après usage prêt à être jeté ;

– La figure 4 représente un mode de réalisation de la serviette périodique selon l'invention : les volets latéraux forment à l'origine partie intégrante d'une pochette de protection dans laquelle est conditionnée la serviette périodique avant emploi ;

– La figure 5 représente la serviette périodique après ouverture de la pochette de protection et dépliement des volets.

– La figure 6 représente la serviette périodique de la figure 5 en position d'emploi, en coupe transversale droite ;

– La figure 7 représente une vue en coupe transversale droite selon la ligne de trace VII-VII de la figure 4 ;

– La figure 8 représente une vue similaire à la figure 7 d'une variante de réalisation ; et

– La figure 9 représente encore une vue en coupe similaire à celle des figures 7 et 8 d'autres variantes de réalisation.

En référence à la figure 1, on a représenté une serviette périodique ou similaire, en particulier pour l'hygiène féminine, pourvue de volets latéraux.

Cette serviette est représentée par le numéro de référence général 1 et comprend un élément tampon proprement dit référencé 2 absorbant les fluides, disposé entièrement à l'intérieur d'une enveloppe 4 dont au moins la partie de réception des fluides (opposée à la partie visible à la figure 1) est perméable aux fluides. En général, la partie de l'enveloppe opposée à la partie de réception des fluides, c'est-à-dire la partie venant se fixer sur le linge de corps qui est la partie visible de la figure 1, est soit réalisée en un matériau étanche aux fluides, soit perméable aux fluides, mais dans ce cas une couche (non représentée ici pour la simplicité du dessin) imperméable aux fluides est interposée entre l'élément tampon 2 et la partie d'enveloppe 4 venant en contact contre le linge de corps. L'élément tampon 2 peut être préformé, par exemple de forme générale biconcave, comme représenté à la figure 1, comme cela est bien connu dans le domaine des serviettes périodiques. Ou bien, l'élément tampon peut être préformé en étant sensiblement rectangulaire et en se terminant par une partie plus étroite à l'arrière comme cela est également bien connu.

Ce dispositif comprend également des volets latéraux 6, 8 s'étendant transversalement depuis la partie centrale de l'élément tampon 2 et de son enveloppe 4. Ces volets latéraux 6, 8 sont d'une dimension suffisante pour pouvoir être rabattus extérieurement autour du linge de corps porté par la personne. Ils peuvent également comporter des moyens de fixation amovibles référencés respectivement 10, 12 pour permettre une fixation des volets extérieurement sur le linge de corps, en permettant ainsi d'obtenir une protection complète du linge de corps.

Ces volets latéraux 6, 8 sont conçus pour former, après emploi de la serviette périodique 2, 4, un conditionnement 20, figure 3 ou sachet jetable, par repliement des volets latéraux 6, 8 sur la serviette périodique 2, 4 repliée également sur elle-même.

Ceci est encore facilité par le fait que, dans l'exemple représenté, la partie de l'enveloppe 4 destinée à venir en contact avec le linge de corps, qui est la face apparente à la figure 1, comporte à ses extrémités antérieure 4a et postérieure 4b des moyens de solidarisation provisoires avec le linge de corps, par exemple des pavés adhésifs 14 et 16, respectivement, ce qui permet d'obtenir avec les moyens de fixation 10, 12 des volets 6, 8 une disposition en croix de l'ensemble des moyens de fixation 10, 12 et de solidarisation 14, 16. Cette disposition en croix est tout à fait avantageuse en ce qu'elle permet, lors du repliement de la serviette périodique, en fait la partie d'élément tampon 2 à l'intérieur de son enveloppe 4, sur elle-même, de fixer l'élément tampon en position repliée grâce aux moyens de solidarisation 14, 16 disposés aux extrémités antérieure et postérieure 4a, 4b, respectivement. Ensuite, on peut aisément rabattre les volets 6, 8 sur l'élément tampon ainsi replié pour aboutir à la configuration de la figure 3 d'un conditionnement ou sachet jetable 20.

Ainsi, avec un tel dispositif, il n'y a donc plus besoin d'un conditionnement séparé pour pouvoir jeter sans problème la serviette usagée.

Les moyens 10, 12 peuvent être disposés, comme cela est représenté sur les figures 1, 2 sur la même face que les pavés 14, 16 pour une fixation des volets directement sur le linge de corps après repliement. Mais ils peuvent être placés différemment. Par exemple, l'un des deux moyens peut être sur la face opposée à celle de 12. La fixation est obtenue alors par chevauchement des volets l'un sur l'autre. Il est possible de ne prévoir qu'un seul moyen 10 ou 12.

En référence à la figure 4, on a représenté un mode de réalisation de serviette avec emballage intégré, conforme à l'invention pour lequel on a utilisé le même numéro de référence qu'à la figure 1, mais augmenté de 100 pour les éléments remplissant la même fonction. Selon l'invention, les volets latéraux 106, 108 font partie intégrante d'une pochette de protection 100 dans laquelle est conditionnée la serviette périodique 102 avant emploi.

La pochette de protection 100 comporte avantageusement des moyens de prédécoupe 122, 124, 126, tels que des lignes de prédécoupe, permettant de libérer les volets latéraux 106, 108 lors de l'ouverture de la pochette de protection 100, comme cela est clairement compréhensible, ce qui permet d'obtenir une serviette périodique ouverte et dépliée, comme représentée à la figure 5. Les moyens de fixation amovibles 110, 112 des volets 106, 108 sont disposés comme à la figure 1, il en est de même des moyens de solidarisation 114, 116 des extrémités antérieure 104a et postérieure 104b de l'enveloppe 104 dans laquelle est disposé l'élément tampon absorbant 102. On obtient ainsi une configuration en croix d'ensemble des moyens de fixation amovibles 110, 112 et des moyens de solidarisation 114, 116.

La partie centrale 128 du dispositif 101 formant serviette périodique est dépourvue d'organes de fixation ou de solidarisation, tels que des pavés de produits adhésifs, ce qui confère une très grande souplesse dans cette zone centrale.

En outre, on observera que dans ce mode de réalisation les moyens de fixation amovibles 110, 112, qui sont provisoirement protégés contre une destruction prématurée par des moyens de protection 130, 132, sont constitués par exemple par des bandes siliconées.

Une fois la pochette de protection 100 ouverte grâce aux prédécoupes 122, 124, 126, et dépliée comme représenté à la figure 5, il suffit d'enlever les moyens de protection 130, 132 pour permettre une fixation des volets 106, 108 extérieurement au linge de corps 140 avec éventuellement un chevauchement des volets 106, 108, comme clairement représenté à la figure 6. Les moyens de fixation amovibles 112 du volet 108 par exemple disposés intérieurement viennent s'appliquer contre le linge de corps 140, tandis que les moyens de fixation amovibles 110 du volet 106 par exemple disposés extérieurement en chevauchement sur le volet 108 viennent se fixer sur le volet lui-même 108. Dans une variante de réalisation, on peut éventuellement supprimer les moyens 112.

Comme cela est représenté sur les figures 4 à 6, les volets 106, 108 sont formés de préférence par une seule bande 142, que l'on voit bien aux figures 5 et 6, en un matériau imperméable aux fluides, par exemple un film de polyéthylène. Naturellement, on peut utiliser tout autre type de matériau convenable. Cette bande 142 est solidarisée par tout moyen à l'enveloppe 104 contenant l'élément absorbant 102. Cela peut être réalisé aisément par collage, par exemple par dépôt d'un ou plusieurs traits de colle 144 qui sont clairement visibles la figure 7.

La fabrication de la pochette de protection 100 avec bande 142 rapportée est réalisée conformément à un procédé dérivé d'un procédé classique de réalisation des emballages individuels pour les serviettes pliées.

Ce procédé consiste à envelopper, en fin de fabrication, les serviettes préalablement pliées en deux ou trois, d'une feuille de polyéthylène ou autre.

L'enveloppage est effectué par mise en place de la serviette sensiblement au milieu de la feuille. On rabat ensuite les bords de cette dernière sur la serviette, l'un sur l'autre. On scelle ces rabats à la portion centrale de la feuille, le long des bords perpendiculaires à l'axe de pliage des rabats. Enfin, on scelle les rabats l'un à l'autre parallèlement à cet axe.

On procède de la même manière si ce n'est qu'on prévoit le dépôt d'un ou plusieurs traits de colle 144 soit sur la feuille 142 destinée à constituer la pochette de protection 100, soit sur l'enveloppe 104 contenant l'élément tampon 102, constituant la serviette périodique, afin d'assurer la solidarisation de ces deux pièces.

En outre, on prévoit les prédécoupes 122, 124, 126 le long des bords scellés de la pochette de protection 100 afin d'assurer une découpe correcte pour réaliser les volets latéraux 106, 108. Au lieu de lignes de prédécoupe, on peut prévoir un scellage non définitif le long des lignes 124, 126. Naturellement encore, ce procédé est modifié pour réaliser les moyens de fixation amovibles 110, 112 des volets 106, 108, par exemple en déposant des pavés d'adhésifs qui sont avantageusement protégés contre toute destruction prématurée par les moyens de protection appropriés 130, 132 tels que des bandes siliconées sur au moins une face.

Diverses variantes de réalisation de cette pochette de protection sont possibles, celles-ci étant représentées en coupe aux figures 7 à 9 selon la même ligne de trace VII-VII de la figure 4. Dans la variante de réalisation des figures 4, 5 et 7, la pochette de protection 100 est réalisée à ce niveau de la soudure, entre les moyens de fixation 110, 112.

Dans la variante de réalisation de la figure 8, les volets latéraux 106, 108 sont solidarisés en chevauchement, par exemple par soudure thermique dans le cas où les volets 106, 108 sont réalisés en une feuille de polyéthylène, par exemple qui est thermosoudable, cette ligne de soudure étant référencée 152.

On peut encore prévoir une variante simplifiée, représentée à la figure 9, selon laquelle un seul des volets latéraux 106, 108, par exemple le volet 106, est pourvu d'un moyen de fixation amovible 110, non protégé qui sert à la fermeture de la pochette de protection 100 par chevauchement du volet 108 extérieurement au volet 106. L'ouverture de la pochette 100 se fait alors par simple action sur le volet 108 Pour le séparer du volet 106. La face interne du volet 108, en regard du moyen 110 peut avoir subi un traitement approprié, connu en soi, par exemple un laquage, pour permettre son détachement sans arracher le moyen 110 du volet 106. On peut ensuite obtenir la fixation des volets 106, 108 entre eux extérieurement au linge de corps 140 par un nouveau chevauchement des volets 106, 108 de manière similaire.

Naturellement, l'invention comprend tous les moyens constituant des équivalents techniques des moyens décrits ainsi que leurs diverses combinaisons. Par exemple, au lieu d'utiliser des pavés de produit adhésif pour réaliser les moyens de fixation amovibles 110, 112, les moyens de solidarisation 114, 116, on peut utiliser des attaches type Velcro (marque déposée).

En outre, les volets latéraux peuvent être formés d'une manière quelconque. On préfère cependant que ces volets latéraux 106, 108 soient rapportés sur l'ensemble enveloppe 104 - élément tampon 102. Ces volets latéraux 106, 108 peuvent être réalisés à partir d'une seule bande 142 en matériau imperméable, afin de protéger en toute sécurité le linge de corps contre tout risque de taches.

Le matériau imperméable utilisé pour former les volets latéraux peut être revêtu d'une matière absorbante afin d'éviter que les gouttes de liquide qui se déposent sur les volets ne tachent le sous-vêtement. Une solution avantageuse consiste à employer pour les volets un complexe pellicule de polyéthylène ou autre matière plastique et non-tissé.

De préférence le non-tissé est constitué de fibres thermofusibles, traitées hémophiles, présentant une douceur au toucher.

Le non-tissé est lié à la pellicule par tout moyen approprié tel que : collage hotmelt, extrusion d'une pellicule de polyéthylène sur le non-tissé ou encore thermoliage par calandrage à chaud.

## Revendications

1. Serviette périodique ou similaire, en particulier pour l'hygiène féminine, comprenant un élément tampon (102) absorbant les fluides disposé entièrement à l'intérieur d'une enveloppe (104) dont au moins la partie de réception des fluides est perméable aux fluides, des volets latéraux (106, 108) étant prévus pour améliorer l'étanchéité latérale, lesdits volets latéraux (106, 108) étant de dimensions suffisantes pour pouvoir être rabattus extérieurement autour du linge de corps, tel que slip, porté par la personne, ces volets latéraux (106, 108) comprenant au moins un moyen (110, 112) permettant la fixation amovible desdits volets latéraux au linge de corps en obtenant ainsi une protection complète dudit linge de corps, lesdits volets latéraux (106, 108) étant conçus de façon à former, après emploi de la serviette périodique, par repliement autour de la serviette périodique elle-même repliée, un conditionnement ou sachet jetable, caractérisée en ce que les volets latéraux (106, 108) sont formés par une bande (142), en matériau imperméable aux fluides, rapportée sur la serviette périodique proprement dite (102-104) et sont également conçus pour former partie intégrante d'une pochette de protection (100) dans laquelle est conditionnée la serviette périodique avant emploi, la pochette (100) étant obtenue d'une part par scellement des volets (106, 108) à la partie centrale de la bande (142) le long des bords perpendiculaires à l'axe de pliage des volets, d'autre part par scellement des volets l'un sur l'autre, parallèlement à cet axe, et comportant des moyens, notamment de prédécoupe, (122, 124, 126) permettant de libérer les volets latéraux (106, 108) lors de l'ouverture de la pochette de protection (100).

2. Serviette périodique selon la revendication 1, caractérisée en ce que les moyens (122, 124, 126) sont constitués par des prédécoupes parallèles aux bords scellés de la pochette (100).

3. Serviette périodique selon la revendication 1 caractérisée en ce que au moins l'un des moyens (122, 124, 126) est constitué par un scellage non définitif des volets à la partie centrale ou entre eux.

4. Serviette périodique selon l'une quelconque des revendications 1 à 3, caractérisée en ce que l'enveloppe précitée (104) dans laquelle est disposé l'élément tampon (102) absorbant les fluides comporte, extérieurement à ses extrémités antérieure (104a) et postérieure (104 b) , des moyens de solidarisation provisoires (114, 116) avec le linge de corps (140) qui servent également au maintien de la serviette périodique en position repliée ; les moyens de fixation (110, 112) des volets (106, 108) étant eux avantageusement disposés vers leurs bords extérieurs, en obtenant ainsi une disposition en croix de l'ensemble des moyens de fixation et de solidarisation.

5. Serviette périodique selon l'une quelconque des revendications 1 à 4, caractérisée en ce que les moyens de fixation amovibles (106, 108) des volets latéraux sont disposés sur un seul (106) des volets (106, 108), en permettant une fixation par chevauchement des extrémités des volets.

6. Serviette périodique selon l'une quelconque des revendications 1 à 5, caractérisée en ce que les moyens de fixation amovibles (110, 112) des volets latéraux (106, 108) sont prévus au niveau de chaque extrémité des volets latéraux sur la paroi extérieure apparente des volets en étant protégés par des moyens de protection contre une destruction prématurée (130, 132), amovibles permettant la formation d'une pochette de protection (100) soit par chevauchement, soit bord à bord.

7. Serviette périodique selon la revendication 6, caractérisée en ce que les moyens de protection contre une destruction prématurée, amovibles (130, 132) peuvent être constitués par une bande siliconée.

8. Serviette périodique selon une des revendications précédentes caractérisée en ce que les volets latéraux (106, 108) sont constitués d'un matériau imperméable aux fluides, revêtu d'une matière absorbante.

**Patentansprüche**

1. Hygienische Binde oder dergleichen, insbesondere für die weibliche Hygiene, mit einem flüssigkeitsabsorbierenden Tamponelement (102), das vollständig innerhalb einer Hülle (104) angeordnet ist, bei der zumindest der flüssigkeitsaufnehmende Teil flüssigkeitsdurchlässig ist, wobei Seitenteile (106,108) zur Verbesserung der seitlichen Abdichtung vorgesehen sind, diese Seitenteile (106,108) ausreichend große Abmessungen haben, um von außen um das von der Person am Körper zu tragende Wäschestück wie ein Slip geschlagen werden zu können, diese Seitenteile (106,108) mindestens ein Mittel (110,112) aufweisen, das eine lösbare Befestigung der Seitenteile am Wäschestück erlauben, um auf diese Weise einen vollständigen Schutz des Wäschestücks zu erzielen, diese Seitenteile (106,108) so ausgebildet sind, daß sie nach Benutzung der hygienischen Binde durch Falten um die ihrerseits gefaltete hygienische Binde ein wegwerfbares Päckchen oder einen wegwerfbaren Beutel bilden, dadurch gekennzeichnet, daß die Seitenteile (106,108) von einem Streifen (142) aus flüssigkeitsundurchlässigem Material gebildet werden, der sich an die eigentliche hygienische Binde (102,104) anschließt, und ebenfalls so ausgebildet sind,daß sie einen integralen Bestandteil einer Schutztasche (100) bilden, in dem die hygienische Binde vor der Benutzung untergebracht ist, wobei die Tasche (100) einerseits durch eine dichte Verbindung der Seitenteile (106,108) mit dem zentralen Abschnitt des Streifens (142) entlang senkrecht zur Faltachse der Seitenteile verlaufenden Rändern und andererseits durch eine dichte Verbindung der Seitenteile miteinander parallel zu dieser Achse gebildet wird und Mittel (122,124,126), insbesondere Schwächungslinien, aufweist, die ein Lösen der Seitenteile (106,108) erlauben, wenn die Schutztasche (100) geöffnet wird.

2. Hygienische Binde nach Anspruch 1, dadurch gekennzeichnet, daß die Mittel (122,124,126) von Schwächungslinien gebildet werden, die parallel zu den Verbindungsrändern der Tasche (100) verlaufen.

3. Hygienische Binde nach Anspruch 1, dadurch gekennzeichnet, daß zumindest eines der Mittel (122,124,126) von einer nicht endgültigen Verbindung der Seitenteile mit dem zentralen Abschnitt oder untereinander gebildet wird.

4. Hygienische Binde nach einem der Ansprüche 1 bis 3, dadurch gekennzeichnet, daß die besagte Hülle (104), in der das flüssigkeitsabsorbierende Tamponelement (102) angeordnet ist, außerhalb seiner vorderen (104a) und hinteren (104b) Enden zur provisorischen Verbindung mit dem Wäschestück (140) dienende Mittel (114,116) aufweist, die gleichzeitig die hygienische Binde in der gefalteten Lage halten, wobei die zur Befestigung der Seitenteile (106,108) dienenden Mittel (110,112) vorzugsweise im Bereich ihrer Außenränder angeordnet sind, um auf diese Weise eine kreuzweise Anordnung des aus den Befestigungs- und Verbindungsmitteln bestehenden Gebildes zu erhalten.

5. Hygienische Binde nach einem der Ansprüche 1 bis 4, dadurch gekennzeichnet, daß die Mittel (106,108) zum lösbaren Befestigen der Seitenteile an einem einzigen (106) der Seitenteile (106,108) angeordnet sind, um eine Befestigung durch Überlappung der Enden der Seitenteile zu ermöglichen.

6. Hygienische Binde nach einem der Ansprüche 1 bis 5, dadurch gekennzeichnet, daß die Mittel (110,112) zum lösbaren Befestigen der Seitenteile (106,108) auf Höhe jeden Endes der Seitenteile an der sichtbaren äußeren Wand der Seitenteile vorgesehen sind, wobei sie durch lösbare Mittel (130, 132) zum Schutz gegen eine vorzeitige Beschädigung geschützt sind, was die Bildung einer Schutztasche (100) entweder durch Überlappung oder durch eine Anordnung Rand gegen Rand ermöglicht.

7. Hygienische Binde nach Anspruch 6, dadurch gekennzeichnet, daß die lösbaren Mittel (130,132) zum Schutz gegen eine vorzeitige Beschädigung von einem Silikonband gebildet werden.

8. Hygienische Binde nach einem der vorhergehenden Ansprüche, dadurch gekennzeichnet, daß die Seitenteile (106,108) aus einem flüssigkeitsundurchlässigen Material bestehen, das mit einem absorbierenden Material beschichtet ist.

**Claims**

1. A sanitary towel or the like, in particular for female hygiene, comprising an absorbent member (102) which absorbs fluid located entirely within an envelope (104) of which at least the part receiving the fluids is permeable to fluids, side flaps (106, 108) being provided to improve lateral liquid-tightness, the said side flaps (106, 108) being of sufficient size to be folded externally around underclothing, such as panties, worn by the person, these side flaps (106, 108) comprising at least one means (110, 112) whereby the said side flaps may be detachably attached to underclothing thus achieving complete protection of the said underclothing, the said side flaps (106, 108) being designed in such a way as to form a disposable package or bag after the sanitary towel has been used by folding them over the sanitary towel which is itself folded, characterised in that the side flaps (106, 108) are formed of a strip (142) of a material which is impermeable to fluids attached to the sanitary towel proper (102-104) and also designed to form an integral part of a protective pouch (100) in which the sanitary towel is packaged before use, the pouch (100) being obtained partly by sealing the flaps (106, 108) to the central part of the strip (142) along edges perpendicular to the fold axis of the flaps, and partly by sealing the flaps to each other, parallel to this axis, and comprising means, in particular cut-outs (122, 124, 126) whereby the side flaps (106, 108) may be released when the protective pouch (100) is opened.

2. A sanitary towel according to claim 1, characterised in that the means (122, 124, 126) comprise cut-outs parallel to the sealed edges of the pouch (100).

3. A sanitary towel according to claim 1, characterised in that at least one of the means (122, 124, 126) comprises non-permanent sealing of the flaps to the central portion or to each other.

4. A sanitary towel according to any one of claims 1 to 3, characterised in that the aforesaid envelope (104) in which the absorbent member (102) absorbing fluids is located comprises means (114, 116) beyond its anterior (104a) and posterior (104b) extremities for attachment to underclothing (140) which also serve to keep the sanitary towel in a folded position; the means for attachment (110, 112) of the flaps (106, 108) being themselves advantageously located towards their outer edges, thus obtaining a cross-shaped arrangement for the set of means of attachment.

5. A sanitary towel according to any one of claims 1 to 4, characterised in that the detachable means of attachment (106, 108) for the side flaps are located on one only (106) of the flaps (106, 108), so that the ends of the flaps may be attached by overlapping.

6. A sanitary towel according to any one of claims 1 to 5, characterised in that the detachable means of attachment (110, 112) for the side flaps (106, 108) are provided at each extremity of the side flaps on the outer visible side of the flaps and are protected by removable means of protection against premature destruction (130, 132) whereby a protective pouch (100) may be formed by overlapping the flaps or placing them edge to edge.

7. A sanitary towel according to claim 6, characterised in that the removable means of protection against premature destruction (130, 132) may comprise a silicone-treated strip.

8. A sanitary towel according to any one of the foregoing claims characterised in that the side flaps (106, 108) comprise a material which is impermeable to fluids coated with an absorbent material.

Fig.1

Fig.2

Fig.3

VII

108

112    132    122

102

126

130

110

124    106

100

Fig.4

Fig.5

114    104

101

102    106

108

112    128    110

142

116

Fig.6

144    102
142
108    140    110    106    112

Fig.7

108    112    132    122    150    130    110    106    100
142    144    102    104

Fig.8

108    152    106    100
102

Fig.9

108    110    106    100
102